# EUROPÄISCHE PATENTSCHRIFT

(11) **EP 1 079 227 B1**
(45) Veröffentlichungstag und Bekanntmachung des Hinweises auf die Patenterteilung: **15.01.2014**
(21) Anmeldenummer: 00114592.9
(22) Anmeldetag: 07.07.2000
(51) Int. Cl.: G01N 21/89, D01H 13/26, G01N 33/36, G01N 21/952

(54) **Verfahren und Vorrichtung zur Detektierung von Fremdkörpern in einem längsbewegten Faden**
Method and device for detecting foreign bodies in a longitudinally moving thread
Méthode et dispositif pour détecter des corps étrangés dans un fil se deplaçant longitudinalement

(30) Priorität: 21.08.1999 DE 19939711
(43) Veröffentlichungstag der Anmeldung: 28.02.2001
(73) Patentinhaber: Saurer Germany GmbH & Co. KG, 42897 Remscheid (DE)
(72) Erfinder: Henze, Herbert, 41063 Mönchengladbach (DE); Birlem, Olav, 41366 Schwalmtal (DE)
(74) Vertreter: Hamann, Arndt

(56) Entgegenhaltungen:
- EP-A- 0 643 294
- WO-A-97/36032
- CH-A- 477 573
- US-A- 4 666 096
- US-A- 5 130 559

## Beschreibung

Die Erfindung betrifft ein Verfahren zur Detektierung von Fremdkörpern in einem längsbewegten Faden nach dem Oberbegriff des Anspruchs 1 und eine Vorrichtung zur Durchführung des Verfahrens.

Das einer Spinnvorrichtung zugeführte Fasermaterial kann Verunreinigungen, Fremdstoffe und Fremdfasern enthalten, die zu sichtbaren unerwünschten Unregelmäßigkeiten im Fertigprodukt oder zu Störungen im Faserverbund und damit zu Fadenbruch bei nachfolgenden Fertigungsprozessen führen können. Dies verursacht Erlöseinbußen bei Vorliegen von Qualitätsminderungen oder Ausschußware beziehungsweise zusätzliche Kosten durch erhöhten Fertigungsaufwand, zum Beispiel bei einer Ersatzfertigung. Es ist daher üblich, Einrichtungen einzusetzen, mit denen derartige Bestandteile aus dem Faden entfernt werden können.

Die EP 0 643 294 offenbart ein Verfahren zur Detektion von Fremdstoffen in einem Faden, bei welchem der Faden mit Licht beaufschlagt, das vom Faden reflektierte Licht gemessen und aus einer Änderung des reflektierten Lichtes auf das Vorhandensein eines Fremdstoffes geschlossen wird. Bei Überschreiten eines Schwellwertes für die helleren und bei Unterschreiten eines Schwellwertes für die dunkleren Fremdstoffe wird ein Fremdstoffsignal generiert und ein Reinigerschnitt ausgelöst.

Aus der EP 0 399 945 ist zur optischen Erkennung von Verunreinigungen, Fremdstoffen und Fremdfasern in textilem Fasermaterial eine Vorrichtung bekannt, die bei sprunghaften Farbänderungen in dem vom Garn reflektierten Licht ein Ausgangssignal zur Auslösung eines Reinigerschnittes generiert.

Die WO 97/36032 beschreibt ein Verfahren nach der Präambel von Anspruch 1.

Farbänderungen wie auch Helligkeitsänderungen treten jedoch nicht nur durch Fremdfasern auf, sondern beispielsweise bei Baumwolle auch durch die im Faden neben den Baumwollfasern enthaltenen Schalen- und Stengelteilchen sowie Blattfragmente. Derartige detektierte Fremdstoffe führen ebenfalls zu Ausgangssignalen des Reinigers und zu Reinigerschnitten. Durch die bereits weitgehende und noch zunehmende Automatisierung des Pflückvorganges bei der Baumwollgewinnung und eine oft damit einhergehende unzureichende Berücksichtigung des optimalen Reifegrades der Baumwolle für den Erntezeitpunkt kann der Anteil pflanzlicher unerwünschter Bestandteile im Fasermaterial einen erheblichen Umfang annehmen. Trotz hohen Reinigungsaufwandes in der Putzerei und der Reinigung beim Kardieren verbleibt ein Teil der pflanzlichen Fremdstoffe im Fasermaterial und wird der Spinnstelle zugeführt. Die Fremdstoffe werden im Faden detektiert, wodurch eine Vielzahl von Reinigerschnitten veranlaßt wird. Die Reinigerschnitte erzwingen jeweils Anspinn- beziehungsweise Fadenverbindungsvorgänge. Eine damit verbundene Vielzahl von Prozessunterbrechungen, in ungünstigen Fällen zum Beispiel erhöht durch mögliche Wiederholungsschaltungen des Anspinnvorganges, senkt den Wirkungsgrad eines Garnherstellungs- oder -verarbeitungsprozesses. Nachteilig dabei ist auch, daß mit jedem Anspinner beziehungsweise jeder Fadenverbindungsstelle zwangsläufig eine mehr oder weniger stark ausgeprägte Dickstelle beziehungsweise eine Schwachstelle verbunden ist und so die Gleichmäßigkeit und die Qualität des Garnes beeinträchtigt wird.

Aufgabe der Erfindung ist, diese vorgenannten Nachteile zu vermindern und den Garnherstellungs- oder verarbeitungsprozess zu verbessern.

Diese Aufgabe wird durch ein Verfahren mit den Merkmalen des Anspruchs 1 und durch eine Vorrichtung mit den Merkmalen des Anspruchs 4 gelöst.

Weitere Ausgestaltungen der Erfindung sind Gegenstand der Unteransprüche.

Gemäß der Erfindung wird eine Einstufung vorgenommen, ob ein Fremdkörper tolerierbar ist oder nicht. Vorzugsweise erfolgt bei der Signalauswertung ein Vergleich der Signale mit vorgebbaren Kriterien, die typische Eigenschaften von als tolerierbar eingestuften Fremdkörpern charakterisieren. Damit sind diese Fremdkörper von anderen Fremdkörpern unterscheidbar. In Abhängigkeit von der Erfüllung dieser Kriterien wird entschieden, ob eine Fadenunterbrechung unterdrückt oder durchgeführt wird. Ein Reinigerschnitt ist eine derartige Fadenunterbrechung. Darunter ist auch das Unterbrechen des Spinnprozesses, zum Beispiel durch Stillsetzen des Rotors, zu verstehen. Durch das Unterdrücken von Fadenunterbrechungen wird nicht bei jedem Signal, das Fremdkörper anzeigt, eine Fadenunterbrechung ausgelöst, und dadurch kann die Zahl der Reinigerschnitte oder anderer Fadenunterbrechungen gesenkt beziehungsweise die von Anspinnern oder anderen Fadenverbindungen hervorgerufenen Nachteile merklich vermindert werden.

Beeinträchtigungen des optischen Eindrucks oder der Festigkeit des Fertigprodukts können dabei vernachlässigbar gering sein, wenn der Fremdkörper, der detektiert wird, zum Beispiel sehr klein ist. Wenn das Entfernen der Fremdkörper in nachfolgenden Herstellungs- oder Bearbeitungsprozessen durch Abstreifen, zum Beispiel an einer Führungsöse, oder durch Herauswaschen, zum Beispiel aus einem Gewebe, möglich ist, tritt gar keine Beeinträchtigung insbesondere der optischen Eigenschaften des Fertigproduktes mehr auf. Für eine Einstufung als tolerierbare Fremdkörper sind entfernbare Fremdkörper besonders geeignet. Vorteilhaft werden die Kriterien so festgelegt, daß sie derartige tolerierbare Fremdkörper charakterisieren.

Eine Fadenunterbrechung bei einem Signal, das einen Fremdkörper anzeigt, wird bevorzugt unterdrückt, wenn mehrere Kriterien gleichzeitig an einer Stelle des Fadens im Detektionsbereich erfüllt sind. Das ist besonders dann der Fall, wenn trotz einer Abweichung der gemessenen Helligkeit um mehr als einen vorbestimmten Wert von einem Sollwert diese Helligkeitsabweichung eine vorbestimmte Ausdehnung in Längsrichtung des Fadens nicht überschreitet sowie eine Variation des Helligkeitssignals im Bereich der Längsausdehnung der Helligkeitsabweichung in Fadenlaufrichtung detektiert wird. Sowohl Ausdehnung wie Helligkeits- beziehungsweise Farbwerte liegen damit im typischen Bereich, der zum Beispiel für Schalenteilchen charakteristisch ist. Durch gleichzeitige Erfüllung mehrerer Kriterien ist eine hohe Sicherheit bei der Erkennung von tolerierbaren Fremdkörpern erreichbar. Die Sicherheit bei der Erkennung von tolerierbaren Fremdkörpern läßt sich noch dadurch erhöhen, daß der Fadendurchmesser zusätzlich laufend detektiert und als zusätzliches Signal ausgewertet wird.

Bevorzugt werden die für die Kriterien erforderlichen vorbestimmten Werte anhand von durch Fremdkörper verunreinigten Garnmustern ermittelt und die ermittelten Werte anschließend abgespeichert. Beispielsweise können die Garnmuster und die darin enthaltenen Fremdkörper nach dem gleichen Verfahren und in der gleichen Vorrichtung untersucht werden, wie sie beim laufenden Produktions- oder Verarbeitungsprozess zur Anwendung kommen. Vergleichende Untersuchungen mit anderen Detektions- und Auswertemitteln wie einer CCD-Kamera können ebenfalls vorgenommen werden. Die abgespeicherten Werte können nicht nur für den aktuellen Detektionsvorgang, sondern auch für spätere Detektionsvorgänge wiederverwendet werden. Die Speicherung der Werte kann zentral in Form einer Datenbank erfolgen und für die Detektion von Fremdkörpern an einer Vielzahl von Arbeitsstellen und Maschinen eingesetzt werden.

Die Kriterien können auch im Verlauf des Herstellungsprozesses automatisch verändert werden. Beispielsweise können langsame Helligkeitsänderungen des Fadens ausgeregelt und die Kriterien beziehungsweise die Grenzwerte angepaßt werden.

Ein besonders vorteilhaftes Verfahren ist die Untersuchung der detektierten Farbspektren. Dabei wird das Farbspektrum des vom Faden reflektierten Lichtes untersucht und dieses Farbspektrum mit gespeicherten Farbspektren von tolerierbaren Fremdstoffen verglichen. Bei Übereinstimmung des untersuchten Farbspektrums mit einem dieser als Kriterium dienenden gespeicherten Farbspektren wird auf das Vorliegen von tolerierbaren Fremdkörpern geschlossen und eine Fadenunterbrechung aufgrund dieses Fremdkörpersignals unterdrückt. Dieses Verfahren ist besonders sicher und gut geeignet für eine Unterscheidung der nicht tolerierbaren Fremdkörper, die zum Beispiel durch einen Reinigerschnitt entfernt werden sollen, von tolerierbaren Fremdkörpern.

Die erfindungsgemäße Vorrichtung weist mindestens eine Lichtquelle und einen Sensor, eine Auswerteeinrichtung sowie Mittel zur Fadenunterbrechung auf. Die Auswerteeinrichtung ist mit einem Datenspeicher gekoppelt. Vorteilhaft sind die vorgebbaren Kriterien in dem Datenspeicher abgelegt. Vorzugsweise erfolgt über die Auswerteeinrichtung ein Vergleich der Signale mit den vorgebbaren Kriterien und in Abhängigkeit von dem Ergebnis des Vergleichs sind die Mittel zur Fadenunterbrechung ansteuerbar. Mit einer derartig ausgebildeten Vorrichtung wird auf besonders einfache und sichere Weise ein tolerierbarer Fremdkörper detektiert und von anderen zu entfernenden Fremdkörpern unterschieden. Fremdkörpersignale, die in Vorrichtungen nach dem bekannten Stand der Technik zu Fadenunterbrechungen führen, lassen sich so unterdrücken.

Ein als CCD-Zeilensensor ausgebildeter Sensor benötigt wenig Raum und liefert aussagekräftige Meßergebnisse. Mit dem CCD-Zeilensensor lassen sich Signalanteile unterdrücken oder separieren, die im Randbereich des Fadens detektiert werden und durchmesserabhängige Werte repräsentieren. Der Einfluß von Durchmesseränderungen des Fadens auf die gemessenen Helligkeitswerte läßt sich so vermeiden. Mit einem CCD-Zeilensensor läßt sich zudem eine außerordentlich hohe Auflösung des Detektionsbereiches auf der Oberfläche des Fadens erzielen. Auf diese Weise können auch kleine Fremdkörper oder die Länge des Fremdkörpers sehr sicher und genau detektiert werden, da auch bei durch kleine Fremdkörper hervorgerufenen Veränderungen noch ausreichend starke Kontraste bei einzelnen Sensorelementen auftreten, während bei einer mit nur einem einzigen Sensorelement vorgenommenen Helligkeitsmessung nur wenig Kontrast geliefert wird und die Empfindlichkeit der Meßvorrichtung dadurch eingeschränkt ist.

In einer Ausbildung der Vorrichtung ist zur Lichterzeugung mindestens eine Lichtquelle mit einem breitbandigen Lichtspektrum vorhanden. Für eine Helligkeitsmessung genügt ein Sensor, für eine Farbspektrumsdetektierung werden mindestens zwei Sensoren eingesetzt oder zum Beispiel ein CCD-Dreizeilensensor, um mit mehr als einen Farbstützpunkt bei der Bestimmung einer Farbe zur Verfügung zu haben. Der CCD-Dreizeilensensor mit Blau-, Grün- und Gelbfilterung eignet sich besonders gut für eine Farbbestimmung mit mehr als einem Farbstützpunkt. In einer alternativen Ausbildung der Vorrichtung sind anstelle einer Lichtquelle mit einem breitbandigen Lichtspektrum mindestens zwei Lichtquellen mit jeweils schmalbandigem Lichtspektrum vorhanden. Auf diese Weise ist ebenfalls ein Arbeiten mit mehr als einem Farbstützpunkt möglich.

Eine Weißlicht-LED als Lichtquelle liefert ein breitbandiges Lichtspektrum und benötigt für den Einbau gegenüber anderen breitbandigen Lichtquellen wenig Bauraum, wie es beim Einsatz in Meßvorrichtungen an Textilmaschinen mit einer Vielzahl von Arbeitsstellen von Vorteil ist.

Die Erfindung ist an Spinnmaschinen, Spulmaschinen und anderen garnverarbeitenden Maschinen wie zum Beispiel Zettelgattern, Webmaschinen und Zwirnmaschinen vorteilhaft einsetzbar.

Die vorliegende Erfindung ermöglicht auf einfache Weise eine wirksame Verminderung der Anspinner beziehungsweise der Fadenverbindungen im Garn. Dies führt zu einer Verbesserung der Garnqualität und zu einer Steigerung des Wirkungsgrades von Produktionsprozessen bei Garnen.

Weitere Einzelheiten der Erfindung sind den Darstellungen der Figuren entnehmbar.

Es zeigt:
- Fig. 1: eine schematische Darstellung einer Ausführungsform der erfindungsgemäßen Vorrichtung,
- Fig. 2: ein Ablaufschema eines erfindungsgemäßen Verfahrens.

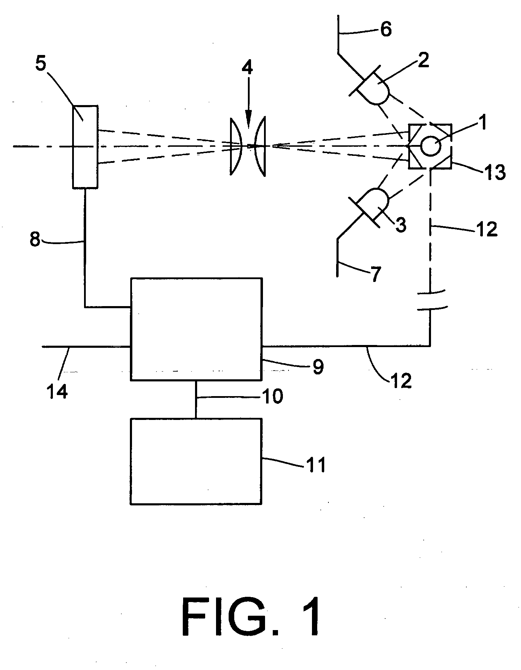

In der schematischen Darstellung der Fig. 1 wird ein längsbewegter Faden 1, der vertikal zur Ebene der Figurendarstellung geführt ist, von zwei als Lichtquelle dienenden Weißlicht-LED 2, 3 mit Licht beaufschlagt. Eine Optik 4 ist derart im Strahlenlauf des vom Faden 1 reflektierten Lichtes angeordnet, daß das reflektierte Licht über die Optik 4 einen Abschnitt des Fadens 1 auf dem Sensor 5 abbildet. Zu weiteren aus Vereinfachungsgründen nicht dargestellten Einzelheiten einer derartigen Meßstelle und des Einsatzortes, zum Beispiel einer Arbeitstelle einer Spinn- oder Spulmaschine, wird auf Ausführungen nach dem Stand der Technik verwiesen, die den bereits genannten Druckschriften EP 0 399 945 und EP 0 643 294 zu entnehmen sind. Zu den beiden Weißlicht-LED 2, 3 führen Leitungen 6, 7, die als Steuer- und Versorgungsleitung dienen. Der Sensor 5 ist über eine Leitung 8 mit der Auswerteeinheit 9 verbunden, wobei die Auswerteeinheit 9 ihrerseits über eine Leitung 10 mit einem Datenspeicher 11 in Verbindung steht. In einer alternativen Ausführung der Vorrichtung bildet der Datenspeicher 11 eine bauliche Einheit mit der Auswerteeinheit 9. Eine weitere Leitung 14 dient dem Datentransfer zwischen der Auswerteeinheit 9 und anderen Datenverarbeitungseinrichtungen oder Produktionselementen, die ebenfalls aus Vereinfachungsgründen nicht dargestellt sind. Die Leitung 12 führt zu einer als Mittel zur Fadenunterbrechung dienenden Schneideinrichtung 13, die vom Faden 1 durchlaufen wird.

Die beiden Weißlicht-LED 2, 3 beaufschlagen den Faden 1 mit einem breitbandigen Lichtspektrum. Der Sensor 5 ist als CCD-Dreizeilensensor ausgebildet. Die in der Fig. 1 beschriebene Anordnung erlaubt eine sichere Bestimmung der Helligkeit beziehungsweise von Helligskeitsabweichungen auf der den Detektionsbereich durchlaufenden Garnoberfläche. Im Datenspeicher 11 sind Daten speicherbar, die typische Eigenschaften von als tolerierbar eingestuften Fremdkörpern charakterisieren. Die Daten können über den Sensor 5 anhand von durch Fremdstoffe verunreinigten Garnmustern ermittelt und die ermittelten Werte anschließend über die Leitungen 8 und 10 und über die Auswerteeinheit 9 im Datenspeicher 11 abgespeichert werden. Über die Leitung 14 können die abgespeicherten Daten aus dem Datenspeicher 11 für die Detektion von Fremdkörpern an einer Vielzahl von Arbeitsstellen und Maschinen abgerufen und eingesetzt werden.

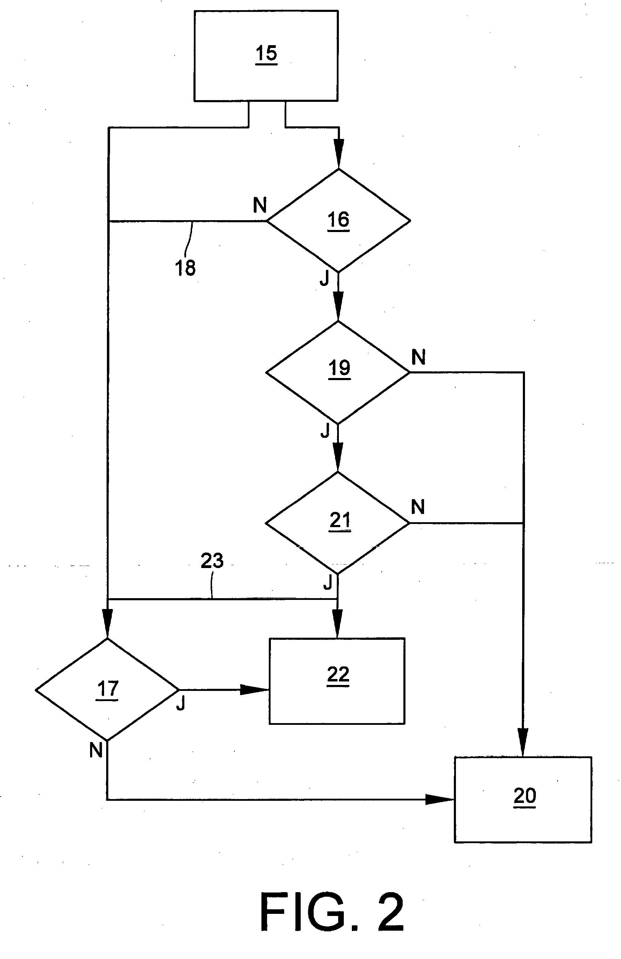

Das in Fig. 2 dargestellte Ablaufschema eines Verfahrens zur Detektierung von Fremdkörpern setzt mit einem mit dem Bezugszeichen 15 gekennzeichneten Verfahrensschritt, einer Signalgenerierung, ein. Im Verfahrensschritt 15 wird das vom Faden reflektierte und vom Sensor 5 detektierte Licht in Signale umgewandelt. Die Signale werden in einem weiteren Schritt, dem Verfahrensschritt 16, einer Helligkeitsprüfung zugeführt. Dabei wird überprüft, ob die gemessene Helligkeit außerhalb eines vorbestimmten Toleranzbereiches eines Sollwertes liegt. Der Toleranzbereich kann in absoluten Werten oder mittels Prozentwerten, die auf den Sollwert bezogen sind, angegeben sein. Es kann auch ein oberer und ein unterer Schwellwert bestimmt werden. Liegt die Helligkeit innerhalb des Toleranzbereiches beziehungsweise zwischen den Schwellwerten, wird entweder die Auswertung zur Detektierung von Fremdkörpern beendet oder alternativ, wie mit der Linie 18 angedeutet, in einem anschließenden Verfahrensschritt 17 eine Überprüfung des Lichtspektrums vorgenommen. Die Ausgänge einzelner Verfahrensschritte sind, wenn ein Kriterium erfüllt ist, mit j ("ja") und, wenn ein Kriterium nicht erfüllt ist, mit n ("nein") gekennzeichnet. Liegt die Helligkeit außerhalb des vorgegebenen Toleranzbereiches, erfolgt nach dem Verfahrensschritt 16 als Verfahrensschritt 19 eine Längenprüfung. Bei der Längenprüfung wird ermittelt, ob die Ausdehnung der Helligkeitsabweichung unter einem vorgegebenen Sollwert liegt. Als Sollwert können beispielsweise 3 mm ausgewählt werden. Der Sollwert kann aber auch andere Werte je nach den gegebenen Erfordernissen annehmen, wie zum Beipiel 1 mm. Liegt die Ausdehnung der Helligkeitsabweichung über dem vorgegebenen Sollwert, wird als Verfahrensschritt 20 ein Reinigerschnitt ausgelöst. Liegt sie unterhalb des Sollwertes, wird eine Variationsprüfung 21 durchgeführt.

Mittels der Variationsprüfung 21 wird untersucht, ob die Helligkeit innerhalb ihrer Ausdehnung Variationen unterliegt beziehungsweise sich im Wert verändert. Ist dies nicht der Fall, wird als Verfahrensschritt 20 ein Reinigerschnitt ausgelöst. Liegt eine Variation vor, findet als Verfahrensschritt 22 eine Unterdrückung des Reinigerschnittes statt. Alternativ kann bei Vorliegen einer Variation des Helligkeitswertes als anschließender Verfahrensschritt 17 eine Überprüfung des Lichtspektrums stattfinden, wie mit der Linie 23 angedeutet. Eine Überprüfung des Lichtspektrums findet somit entweder direkt nach der Signalgenerierung im Anschluß an den Verfahrensschritt 15 oder aber alternativ nach Feststellen eines Helligkeitswertes außerhalb der vorgegebenen Toleranz und direkt nach Feststellen einer Variation des Helligkeitswertes innerhalb der Ausdehnung der Helligkeitsabweichung. Bei einer Überprüfung des Lichtspektrums wird das Farbspektrum des vom Faden reflektierten Lichtes bestimmt. Dieses Farbspektrum wird mit im Datenspeicher 11 gespeicherten Daten von Farbspektren, die tolerierbare Fremdstoffe charakterisieren, verglichen. Stimmt das Farbspektrum mit mindestens einem als Kriterium dienenden gespeicherten Farbspektrum überein, erfolgt als darauf folgender Verfahrensschritt 22 eine Unterdrückung des Reinigerschnitts.

Die in den Verfahrensschritten 15, 16, 17, 19 und 21 durchgeführten Funktionen beziehungsweise Überprüfungen können auch gleichzeitig und die Auswertung parallel dazu oder anschließend erfolgen.

Als Kriterium dienende Daten lassen sich zum Beispiel dadurch bestimmen, daß die erforderlichen Werte anhand von durch Fremdstoffe verunreinigten Garnmustern ermittelt und anschließend gespeichert werden. Eine weitere Auswahl kann dadurch vorgenommen werden, daß die Daten nur Fremdkörper charakterisieren, die in nachfolgenden Verarbeitungsprozessen entfernt werden können. Die Werte können auch manuell über eine nicht dargestellte Tastatur eingegeben oder über die Leitung 14 eingespeist werden. Die Auswahl und Festlegung von Kriterien oder Werten kann auch aus der Erfahrung der Bedienungsperson erfolgen oder durch sie beeinflußt werden.

Mit dem Erfindungsgegenstand ist in einer Vielzahl von Fällen eine Unterdrückung einer Fadenunterbrechung, wie zum Beispiel die Unterdrückung eines Reinigerschnittes, und somit die Senkung einer unerwünscht hohen Anzahl von Anspinnern oder Fadenverbindungen möglich. Dadurch läßt sich die Garnqualität erhöhen und der Wirkungsgrad von Produktionsprozessen steigern. Dies bedeutet eine Verbesserung des Garnherstellungs- oder verarbeitungsprozesses.

## Patentansprüche

1. Verfahren zur Detektierung von Fremdkörpern in einem längsbewegten Faden (1), wobei Licht auf den Faden (1) gerichtet und von dessen Oberfläche reflektiert wird, vom Faden (1) reflektiertes Licht von einem Sensor (5) detektiert und in Signale umgewandelt wird sowie anschließend eine Auswertung der Signale stattfindet und abhängig vom Ergebnis dieser Auswertung eine Fadenunterbrechung ausgelöst wird,
wobei eine Einstufung vorgenommen wird, ob ein Fremdkörper tolerierbar ist oder nicht,
wobei Kriterien vorgegeben werden, die typische Eigenschaften von tolerierbaren Fremdkörpern zur Unterscheidung von anderen Fremdkörpern charakterisieren,
wobei bei der Signalauswertung ein Vergleich der Signale mit den vorgebbaren Kriterien erfolgt
und in Abhängigkeit von der Erfüllung dieser Kriterien entschieden wird, ob eine Fadenunterbrechung unterdrückt oder durchgeführt wird
**dadurch gekennzeichnet,**
**dass** die Kriterien so festgelegt werden, dass sie Fremdkörper charakterisieren, deren Entfernung in nachfolgenden Verarbeitungsprozessen durch Abstreifen oder durch Herauswaschen möglich ist,
und **dass** die Kriterien so festgelegt werden, dass eine Fadenunterbrechung unterdrückt wird, wenn
- trotz einer Abweichung der gemessenen Helligkeit um mehr als einen vorbestimmten Wert von einem Sollwert diese Helligkeitsabweichung eine vorbestimmte Ausdehnung in Längsrichtung des Fadens (1) nicht überschreitet
- und eine Variation des Helligkeitssignals im Bereich der Längsausdehnung der Helligkeitsabweichung detektiert wird.

2. Verfahren nach einem der vorhergehenden Ansprüche, **dadurch gekennzeichnet, dass** die für die Kriterien erforderlichen vorbestimmten Werte anhand von durch Fremdkörper verunreinigten Garnmustern ermittelt und anschließend abgespeichert werden.

3. Verfahren nach einem der vorhergehenden Ansprüche, **dadurch gekennzeichnet, dass** das Farbspektrum des vom Faden (1) reflektierten Lichtes bestimmt wird und dieses Farbspektrum mit gespeicherten Farbspektren von tolerierbaren Fremdkörpern verglichen wird und bei Übereinstimmung mit einem dieser als Kriterium dienenden gespeicherten Farbspektren die Fadenunterbrechung unterdrückt wird.

4. Vorrichtung zur Durchführung des Verfahrens nach Anspruch 1, die eine Lichtquelle, einen Sensor (5), eine Auswerteeinrichtung (9) sowie Mittel zur Fadenunterbrechung aufweist, wobei die Auswerteeinrichtung (9) mit einem Datenspeicher (11) gekoppelt ist, wobei in dem Datenspeicher (11) die vorgebbaren Kriterien abgelegt sind, wobei die Kriterien so festgelegt sind, dass sie Fremdkörper charakterisieren, deren Entfernung in nachfolgenden Verarbeitungsprozessen durch Abstreifen oder durch Herauswaschen möglich ist, wobei über die Auswerteeinrichtung (9) ein Vergleich der Signale mit den vorgebbaren Kriterien erfolgt und in Abhängigkeit von dem Ergebnis des Vergleichs die Mittel zur Fadenunterbrechung ansteuerbar sind und eine Variation des Helligkeitssignals im Bereich der Längsausdehnung der Helligkeitsabweichung detektierbar ist wobei die Kriterien so festgelegt sind, dass eine Fadenunterbrechung unterdrückt wird, wenn trotz einer Abweichung der gemessenen Helligkeit um mehr als einen vorbestimmten Wert von einem Sollwert diese Helligkeitsabweichung eine vorbestimmte Ausdehnung in Längsrichtung des Fadens nicht überschreitet und eine Variation des Helligkeitssignals im Bereich der Längsrichtung der Helligkeitsabweichung detektiert wird.

5. Vorrichtung nach Anspruch 4, **dadurch gekennzeichnet, dass** der Sensor (5) ein CCD-Zeilensensor, insbesondere ein CCD-Dreizeilensensor mit Blau-, Grün- und Gelbfilterung, ist.

6. Vorrichtung nach einem der Ansprüche 4 oder 5, **dadurch gekennzeichnet, dass** die Lichtquelle eine Weißlicht-LED (2, 3) ist.

## Claims

1. Method for detecting foreign bodies in a longitudinally travelling yam (1), in which light is directed onto the yam (1) and is reflected from its surface, light reflected from the yarn (1) is detected by a sensor (5) and converted into signals and after this the signals are evaluated and depending on the result of this evaluation the yam is interrupted,
a grading being carried out to determine whether a foreign body is tolerable or not,
criteria being defined which characterise typical properties of tolerable foreign bodies to distinguish them from other foreign bodies, during the signal evaluation a comparison is made of the signals with the predefinable criteria,
and depending on whether these criteria have been met or not a decision is taken whether to suppress or perform the yam interruption, **characterised in that** the criteria are defined so that they characterise foreign bodies which can be removed in subsequent processing stages by stripping off or washing out,
and **in that** the criteria are defined so that the yam interruption is suppressed if
- despite a deviation of the measured brightness from the reference value by more than a predetermined value said brightness deviation does not exceed a predetermined extension in the longitudinal direction of the yam (1),
- and a variation in the brightness signal is detected in the region of the longitudinal extension of the brightness deviation.

2. Method according to one of the preceding claims, **characterised in that** the predetermined values necessary for the criteria are determined with reference to yam samples polluted with in foreign bodies and are then saved.

3. Method according to one of the preceding claims, **characterised in that** the colour spectrum of the light reflected by the yam (1) is determined and said colour spectrum is compared with saved colour spectra of tolerable foreign bodies and the yarn interruption is suppressed if this coincides with one of these colour spectra used as the criterion.

4. Device for performing the method according to claim 1, which comprises a light source, a sensor (5), an evaluation device (9) and means for interrupting the yam, in which the evaluation device (9) is connected to a data memory (11), the predeterminable criteria are stored in the data memory (11), the criteria are set so that they characterise foreign bodies which can be removed in subsequent processing stages by stripping off or washing out,
by means of the evaluation device (9) the signals are compared with the predeterminable criteria and depending on the result of the comparison the means for interrupting the yam can be controlled and a variation in the brightness signal can be detected in the region of the longitudinal extension of the brightness deviation,
and wherein the criteria are defined so that a yam interruption is suppressed if despite a deviation of the measured brightness by more than a predefined value from a reference value said brightness deviation does not fall below a predetermined extension in the longitudinal direction of the yarn and a variation in the brightness is detected in the region of the longitudinal extension of the brightness deviation.

5. Device according to claim 4, **characterised in that** the sensor (5) is a CCD line sensor, in particular a CCD three-line sensor with blue, green and yellow filtering.

6. Device according to one of claims 4 or 5, **characterised in that** the light source is a white light LED (2, 3).

## Revendications

1. Procédé assigné à la détection de corps étrangers dans un fil (1) animé d'un mouvement longitudinal, de la lumière étant dirigée vers ledit fil (1) et étant réfléchie par la surface de ce dernier, de la lumière réfléchie par ledit fil (1) étant détectée par un capteur (5) et étant convertie en des signaux, après quoi une évaluation desdits signaux est effectuée et une interruption du fil est déclenchée en fonction du résultat de cette évaluation,
sachant qu'il est procédé à une classification visant à établir si un corps étranger peut, ou non, être toléré,
sachant que des critères, caractérisant des propriétés typiques de corps étrangers tolérables, sont prédéfinis en vue de la différenciation d'avec d'autres corps étrangers, sachant qu'une comparaison des signaux, avec lesdits critères prédéfinissables, est effectuée au cours de l'évaluation desdits signaux
et sachant qu'une décision, visant à établir si une interruption du fil est entravée ou exécutée, intervient en fonction de la satisfaction à ces critères,
**caractérisé par le fait**
**que** les critères sont fermement établis de façon telle qu'ils caractérisent des corps étrangers dont l'élimination est possible au cours de processus ultérieurs de traitement, par raclement ou par lavage,
et **que** lesdits critères sont fermement établis de telle sorte qu'une interruption du fil soit entravée lorsque,
- en dépit d'un écart de la luminosité mesurée, dépassant une valeur de consigne d'une valeur prédéterminée, cet écart de luminosité n'excède pas une étendue prédéterminée dans la direction longitudinale du fil (1)
- et une variation du signal de luminosité est détectée dans la région de l'étendue longitudinale dudit écart de luminosité.

2. Procédé selon l'une des revendications précédentes, **caractérisé par le fait que** les valeurs prédéterminées, requises pour les critères, sont instaurées à l'appui de modèles de fils souillés par des corps étrangers, et sont ensuite mémorisées.

3. Procédé selon l'une des revendications précédentes, **caractérisé par le fait que** le spectre des couleurs de la lumière réfléchie par le fil (1) est déterminé, et ce spectre des couleurs est comparé à des spectres de couleurs mémorisés de corps étrangers tolérables, l'interruption du fil étant entravée en cas de coïncidence avec l'un de ces spectres de couleurs mémorisés, servant de critère.

4. Dispositif dévolu à la mise en oeuvre du procédé selon la revendication 1, comprenant une source de lumière, un capteur (5), un système d'évaluation (9), ainsi que des moyens dédiés à l'interruption du fil, ledit système d'évaluation (9) étant couplé à une mémoire de données (11), les critères prédéfinissables étant consignés dans ladite mémoire de données (11), lesdits critères étant fermement établis de façon telle qu'ils caractérisent des corps étrangers dont l'élimination est possible au cours de processus ultérieurs de traitement, par raclement ou par lavage, une comparaison des signaux avec lesdits critères prédéfinissables étant effectuée par l'intermédiaire dudit système d'évaluation (9), lesdits moyens d'interruption du fil pouvant être activés en fonction du résultat de ladite comparaison, une variation du signal de luminosité pouvant être détectée dans la région de l'étendue longitudinale de l'écart de luminosité, et lesdits critères étant fermement établis de telle sorte qu'une interruption du fil soit entravée lorsque, en dépit d'un écart de la luminosité mesurée, dépassant une valeur de consigne d'une valeur prédéterminée, cet écart de luminosité n'excède pas une étendue prédéterminée dans la direction longitudinale du fil, et une variation dudit signal de luminosité est détectée dans la région de l'étendue longitudinale dudit écart de luminosité.

5. Dispositif selon la revendication 4, **caractérisé par le fait que** le capteur (5) est un capteur linéaire à transfert de charge, en particulier un capteur à transfert de charge en trois lignes à filtrages bleu, vert et jaune.

6. Dispositif selon l'une des revendications 4 ou 5, **caractérisé par le fait que** la source de lumière est une diode électroluminescente (2, 3) à lumière blanche.
